# EUROPEAN PATENT SPECIFICATION

(11) **EP 3 395 260 B1**
(45) Date of publication and mention of the grant of the patent: **29.07.2026**
(21) Application number: 16879047.5
(22) Date of filing: 26.12.2016
(51) Int. Cl.: A61B 17/04, A61B 17/12, A61B 17/062, A61B 17/00

(54) **ENDOSCOPIC SUTURE LIGATION TOOL**
ENDOSKOPISCHES NAHTLIGATIONSWERKZEUG
OUTIL DE LIGATURE DE SUTURE ENDOSCOPIQUE

(30) Priority: 24.12.2015 JP 2015251218; 24.12.2015 JP 2015251219; 21.12.2016 JP 2016248490
(43) Date of publication of application: 31.10.2018
(73) Proprietor: Zeon Corporation, Tokyo 100-8246 (JP)
(72) Inventor: SAKAKI, Kohei, Tokyo 100-8246 (JP); INOUE, Koichi, Tokyo 100-8246 (JP); SHINAGAWA, Hiroki, Tokyo 100-8246 (JP)
(74) Representative: Maiwald GmbH
(86) International application number: PCT/JP2016/088727
(87) International publication number: WO 2017/111163

(56) References cited:
- EP-A1- 1 859 747
- JP-A- 2005 329 239
- JP-A- 2012 024 276
- JP-A- 2013 233 433
- JP-A- 2013 509 254
- JP-B2- 5 294 181
- US-A- 5 855 586
- US-A- 5 989 252
- US-A1- 2003 167 062
- US-A1- 2006 253 128

## Description

### TECHNICAL FIELD

The present invention relates to an endoscopic suture ligation tool for ligating a suture of an incision or so formed to a lumen wall of a gastrointestinal tract or so during natural orifice translumenal endoscopic surgery.

### BACKGROUND ART

Natural orifice translumenal endoscopic surgery (NOTES) is recently widely known as a minimally invasive surgery which does not make a cut to a body surface. On the contrary to the conventional surgical operation which reaches to a body cavity by making an incision to the body surface, an endoscope is used to enter the body cavity such as abdominal cavity or so from mouth or anus via gastrointestinal tract, or via vagina or urinary bladder or so. The possibilities to be used for various procedures have been reported such as diagnostic procedure such as intraperitoneal observation and liver biopsy or so, appendectomy, cholecystectomy, tubal ligation, ovariectomy, and gastrojejunostomy or so.

For such natural orifice translumenal endoscopic surgery, as a suturing device to suture an incision (including pierced part and defective part) formed to the lumen wall of gastrointestinal tract or so, those disclosed in a patent document 1 is proposed. In this device, one end of the suture passed across a bifurcate part of an arm provided at an outside (body cavity side) of the lumen by penetrating through the incision is engaged with the tip of a needle pierced from the inside of the lumen to the lumen wall of one side of the incision, then the needle is removed, thereby one end of the suture is guided to the inside of the lumen by penetrating through the lumen wall. Then, similarly other end of suture is engaged to the tip of the needle pierced from the inside of the lumen to the lumen wall of other side of the incision, then the needle is removed, thereby other end of the suture is guide to the inside of the lumen by penetrating through the lumen wall. Then, the both ends of the suture guided to the inside of the lumen are tightened using a ligation tool thereby ligation is done.

As the ligation tool used for ligating the suture, as discloses in the patent document 1 which is the ligation member, both ends of a wire member is connected and formed in a loop form, and also a middle part of the wire member is made closer to each other so that the middle part is approximately parallel, and a tube is fit in a slidable manner to the contacted part. The loop formed on the one side against the tube is a ligation loop for ligating the suture, and the other loop is a linking loop connected to the ligation device. Using the ligation device, the linking loop is pulled with respect to the tube, and the tube is slid towards the ligation loop side, thereby the ligation loop where the both ends of the suture are passed through is pulled to the inside of the tube together with the suture, and locked by the pressure of the inner wall of the tube. Thereby, the suture is tightened and the incision is closed, thus ligation is done.

However, for the conventional ligation tool, when the linking loop is pulled with respect to the tube so that the ligation loop is pulled into the tube, in some case the ligation loop slips out from the tube because the loop member is pulled too much against the tube, and the loop member and the tube were separated in some cases. Thus, in such case, the ligation was unable to be done in good condition.

### PRIOR ART

US5855586A describes that a ligature comprises a ligation loop located on the distal side of the ligature, a stopper through which the ligation loop is passed in a united state, a hooked loop located proximally of the stopper, and a restriction member formed so that a plasto-elastic wire forming the ligation loop can penetrate through the restriction member. The ligation loop and hooked loop are formed with plasto-elastic wires made of a synthetic resin. The cylindrical stopper is press-fitted on the part of the ligation loop folded in two, and the hooked loop is formed behind the stopper. The ligation loop is provided with the restriction member formed with a plasto-elastic tubular member with the wire of the ligation loop passed through the restriction member.
US2006/253128A1 describes that when a living tissue of a ligation target is ligated by means of a snare wire, the snare wire is brought into contact with the living tissue of a ligation target, on a large contact face by means for preventing living tissue cut-in of a ligation target of at least one of a distal end chip and a rear end ring of a loop section of the snare wire, thereby preventing the living tissue of a ligation target from cutting into a contact face of at least one of the distal end chip and rear end ring of the loop section. EP1859747A1 describes that a medical suturing and ligating apparatus is provided which enable to cut a suturing and ligating member such as a ligating wire in a simple manner while preventing a holding member from being removed from a flexible sheath. It is described that when a slider pulls a ligating wire toward the proximal end of an inner sheath so as to suture or ligate a living tissue with the ligating wire when a wire holding member is in abutting contact with the distal end of the inner sheath, a fixing screw engages with an engaging recessed portion.
JP2013509254A describes apparatus and methods for treating tissue by applying a force to the tissue. It is described that the apparatus comprises a deployable segment having proximal and distal regions, a main body extending therebetween, and a loop member formed at the distal region of the deployable segment. It is described that a cannula having a bore is dimensioned to circumferentially surround at least a portion of the main body at a location proximal to the loop member, and a spring member is disposed between the cannula and the loop member. It is described that the proximal end of the spring member is affixed to the cannula, and the distal end of the spring member is movable to apply a compressive force to adjust the size of the opening of the loop member and compress tissue disposed within the opening of the loop member. JP2005329239A describes that the ligation treating device has a ligating string, a silicone tube which is a fixing member, and a flexible wire material which is a ligation cancelling member. It is described that the ligating string has a distal end portion and a proximal end portion and ligates the biological tissue. It is described that the silicone tube is arranged so as to freely move back and forth to the ligating string and fixed to the ligating string by friction force so as to maintain in a condition where the biological body is ligated by the ligating string. It is described that the flexible wire material is secured to the ligating string, the ligation condition between the ligating string and the silicone tube is cancelled by moving the ligating string to the front end side relative to the silicone tube.
[Patent document 1] JP Patent No.5294181

### SUMMARY OF INVENTION

### PROBLEM TO BE SOLVED BY THE INVENTION

The present invention is done in view of such circumstances, and the object is to provide the endoscopic suture ligation tool capable of carrying out the ligation of the suture in good condition.

### MEANS FOR SOLVING THE PROBLEM

The invention and its scope of protection is defined by the appended independent claim. Embodiments of the invention are defined by the appended dependent claims.

The endoscopic suture ligation tool according to the present invention includes endoscopic suture ligation tool having,
a ligation loop for passing a suture through,
a linking loop connected to a ligation device,
a connecting part connecting the ligation loop and the linking loop,
a tube fitted in a slidable manner over the connecting part such that the ligation loop is on one side of the tube and the linking loop is on the opposite side of the tube, wherein the tube is configured to be slid towards the ligation loop side or the linking loop is configured to be pulled with respect to the tube so that the entire ligation loop is pulled into a lumen of the tube with the suture, and and
a locking means provided to the ligation loop, the linking loop or the tube to lock a part of the ligation loop so that the ligation loop is not released from an inside of the tube when pulling the entire ligation loop into the lumen of the tube.

The endoscopic suture ligation tool according to the present invention has the locking means to lock the ligation loop at the inside of the tube so that it does not slip out from the inside of the tube. Therefore, the ligation of the suture can be done in good condition because this prevents the ligation loop from slipping out of the inside of the lumen which is caused by pulling in the ligation loop too much when the linking loop is pulled with respect to the tube in order to pull in the ligation loop to the inside of the tube.

The endoscopic suture ligation tool according to the present invention can have the connecting part which is a columnar part, and the ligation loop is connected to one end of the connecting part and the linking loop is connected to the other end of the connecting part. The ligation loop and the linking loop can be suppressed from deforming, and the shape can be stabilized.

In the endoscopic suture ligation tool according to the present invention, the tube may fit to a middle part of a loop member made of a flexible endless wire member so that the loop member inside of the tube is facing against each other,
the ligation loop may be formed of the loop member at one side of the tube, and the linking loop may be formed of the loop member at the other side of the tube, and
the connecting part may be a part where the tube is being fit.

In the endoscopic suture ligation tool according to the present invention, the locking means may include a caulking member fit to the tube so that a part of the lumen of the tube is narrower.

In the endoscopic suture ligation tool according to the present invention, the locking means may include a stopper comprising,
a plate part having a first through hole where a part of the linking loop passes through in a slidable manner and a second through hole where other part of the linking loop passes through, and
a projection part formed integrally with the plate part at about a center of the plate part while projecting out to the tube side so that the projection part can be inserted to the lumen.

In the endoscopic suture ligation tool according to the present invention, the locking means may include a stopper comprising,
a plate part having a larger diameter than an inner diameter of the tube, and
a connection loop having approximately U shape and capable of being inserted into the lumen of the tube wherein one end of the connection loop is integrally connected to a part of the plate part, an intermediate part of the connection loop is passed through the ligation loop, and the other end of the connection loop is integrally connected with other part of the plate part.

In the endoscopic suture ligation tool according to the present invention, the locking means may include a stopper ring made of endless wire member wound around the tube, and the stopper ring passes through the linking loop while running along a side face of one side of the tube, and the stopper ring passes through the ligation loop while running along the other side of the tube.

In the endoscopic suture ligation tool according to the present invention, the locking means may include a projection part integrally formed with the ligation loop.

In the endoscopic suture ligation tool according to the present invention, the locking means may include a larger diameter part formed integrally with the ligation loop and has larger diameter than a diameter of the other part of the ligation loop.

The endoscopic suture ligation tool according to the present invention, the locking means may include a locking part having larger size than an inner diameter of the tube, and provided to an end part opposite of the connecting part of the ligation loop. The locking part does not enter the lumen of the tube and contact with the end face of the tube, thus the ligation loop is securely prevented from slipping out from the lumen of the tube.

### BRIEF DESCRIPTION OF DRAWING

[Fig.1] Fig.1 is a perspective view showing a constitution of the endoscopic suture ligation tool of the first embodiment of the present invention.
[Fig.2] Fig.2 is a cross section view showing a constitution during the ligation of the endoscopic suture ligation tool of the first embodiment of the present invention, and the constitution around there.
[Fig.3] Fig.3 is a perspective view showing a constitution of the endoscopic suture ligation tool of the second embodiment of the present invention.
[Fig.4] Fig.4 is a cross section view showing a constitution during the ligation of the endoscopic suture ligation tool of the second embodiment of the present invention, and the constitution around there.
[Fig.5] Fig.5 is a perspective view showing a constitution of the endoscopic suture ligation tool of the third embodiment of the present invention.
[Fig.6] Fig.6 is a cross section view showing a constitution during the ligation of the endoscopic suture ligation tool of the third embodiment of the present invention, and the constitution around there.
[Fig.7] Fig.7 is a perspective view showing a constitution of the endoscopic suture ligation tool of the fourth embodiment of the present invention.
[Fig.8] Fig.8 is a cross section view showing a constitution during the ligation of the endoscopic suture ligation tool of the fourth embodiment of the present invention, and the constitution around there.
[Fig.9] Fig.9 is a perspective view showing a constitution of the endoscopic suture ligation tool of the fifth embodiment of the present invention.
[Fig.10] Fig.10 is a perspective view showing a constitution of the endoscopic suture ligation tool of the sixth embodiment of the present invention.
[Fig.11] Fig.11 is a plan view showing a constitution of the endoscopic suture ligation tool of the seventh embodiment of the present invention.
[Fig.12] Fig.12 is a plan view showing the tube of the endoscopic suture ligation tool of the seventh embodiment of the present invention before fitting.
[Fig.13] Fig.13 is a partial cross section view showing the ligation of the suture by the endoscopic suture ligation tool of the seventh embodiment of the present invention.
[Fig.14] Fig.14 is a figure showing a constitution of a suturing device of an embodiment of the present invention, and steps of suturing (step 1).
[Fig.15] Fig.15 is a figure showing steps of the suturing of an embodiment of the present invention (step 2).
[Fog.16] Fig.16 is a figure showing steps of the suturing of an embodiment of the present invention (step 3).
[Fig.17] Fig.17 is a figure showing steps of the suturing of an embodiment of the present invention (step 4).
[Fig.18] Fig.18 is a figure showing steps of the suturing of an embodiment of the present invention (step 5).
[Fig.19] Fig.19 is a figure showing steps of the suturing of an embodiment of the present invention (step 6).
[Fig.20] Fig.20 is a figure showing steps of the suturing of an embodiment of the present invention (step 7).
[Fig.21] Fig.21 is a figure showing steps of the suturing of an embodiment of the present invention (step 8).
[Fig.22] Fig.22 is a figure showing steps of the suturing of an embodiment of the present invention (step 9).
[Fig.23] Fig.23 is a figure showing steps of the suturing of an embodiment of the present invention (step 10).
[Fig.24] Fig.24 is a figure showing steps of the suturing of an embodiment of the present invention (step 11).
[Fig.25] Fig.25 is a plan view showing the ligation device of an embodiment of the present invention.

Hereinafter, an embodiment of the present invention will be described by referring to the figures.

### (First Embodiment)

### (Endoscopic suture ligation tool)

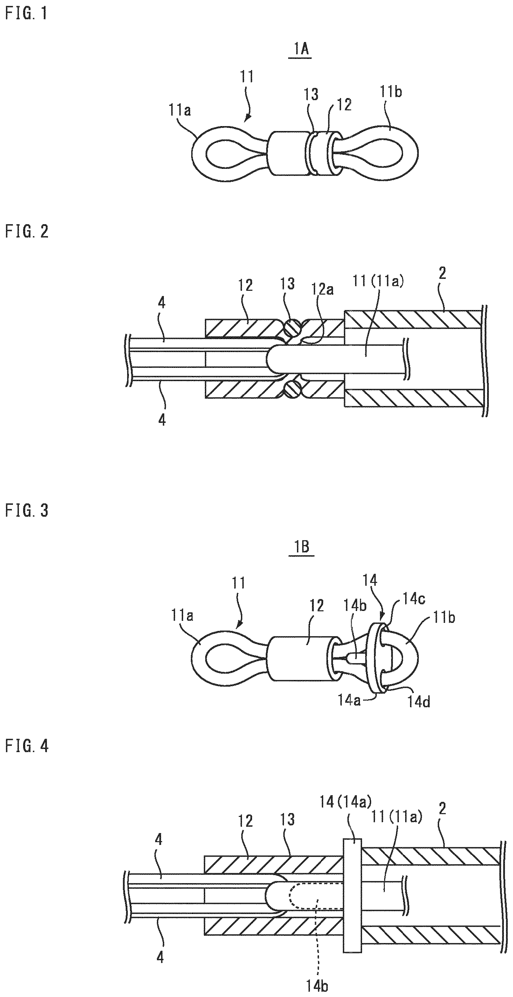

Fig.1 is a perspective view showing the constitution of the endoscopic suture ligation tool according to a first embodiment of the present invention, and Fig.2 is a cross section view showing the endoscopic suture ligation tool during the ligation, and the area around there. The endoscopic suture ligation tool 1A has a main body part (loop member) 11 and tube (tightening tube) 1, wherein the main body part 11 is made by binding both ends of a flexible wire member and molding into a loop form (endless form), and the tube 12 is made of flexible material and has approximately cylinder from and fits to a middle part of the main body part 11 in a slidable manner so that the middle part of the main body part 11 is approximately parallel and close to each other.

A ligation loop 11a for ligating a suture 4 (which will be described in below) is formed at one side (left side of Fig.1) of the main body part 11 against tube 12, and the linking tube 11b is the other side (right side of Fig.1) which will be connected to a ligation device 2 (which will be described in below). The part which connects the ligation loop 11a and the linking loop 11b is the connecting part of the present invention (where the tube 12 is fit). As the material of the main body part 11, there is no particular limitation as long as the material has appropriate flexibility, and in the present embodiment polyamide resin is used. Also, as the material of the tube 12, there is no particular limitation as long as the material has appropriate elasticity, and silicone elastomer is used in the present embodiment.

The both ends of the suture 4 are tied and passed through the ligation loop 11a, then using the ligation device 2, the tube 12 is slid towards the ligation loop 11a side (or the linking loop 11b is pulled with respect to the tube 12) so that the ligation loop 11a is pulled into the tube 12. Thereby, the ligation loop 11a is pulled into the tube 12 together with the suture 4, and the pulling is stopped when a turning point which is the tip part of the ligation loop 11a reaches to the middle part of the tube 12, thereby the ligation of the suture 4 is done.

Note that, when the ligation loop 11a is pulled into the tube 12 together with the suture 4, although it is not shown in Fig.2, the tube 12 expands outwards due to the pressure from the suture 4 and the turning point of the ligation loop 11a, and the ligation loop 11a and the suture 4 are firmly held in the tube 12 by the restoring force (tightening force) of the tube 12.

The wire diameter of the wire member constituting the main body part 11 is 0.45 mm in the present embodiment. The size in axial direction of the tube 12 is 5.0 mm in the present embodiment. The outer diameter of the tube 12 is 1.6 mm in the present embodiment. The inner diameter of the tube 12 is 0.3 mm in the present embodiment.

The first embodiment has a caulking member (ring form member) 13 fit to the tube 12 as the locking means for locking the tip (turning point) of the ligation loop 11a in the tube 12 so that the tip of the ligation loop 12 does not slip out from the lumen of the tube 12, and the caulking member 13 is formed so that a narrower diameter part 12a narrower than the lumen of tube is formed to part of the lumen of the tube 12. As the caulking member 13, O ring of approximately circular ring form or C ring made of metal or so having the outer diameter about the same as the outer diameter of the tube 12 or narrower than this is used, and the tube 12 is inserted to the inside and caulking is done from the outside with a pressure; thereby the caulking ring can be installed. The inner diameter of the narrower diameter part 12a is 0.15 mm in the present embodiment.

The turning point (tip part) of the ligation loop 11a of the main body part 11 has larger size than the middle part which is arranged approximately parallel to each other of the main body part 11, and the suture 4 passes through the inside of the turning point when ligating. Hence, when the tube 12 is slid to the ligation loop 11a side and the ligation loop 11a is pulled into the tube 12 together with the suture 4, the turning point is interfered and locked at the narrower diameter part 12a formed at the lumen of the tube 12 by the caulking member 13. Thereby, the ligation loop 11a is prevented or suppressed from slipping out of the tube 12 which is caused by pulling the ligation loop 11a too much against the tube 12.

### (Ligation device)

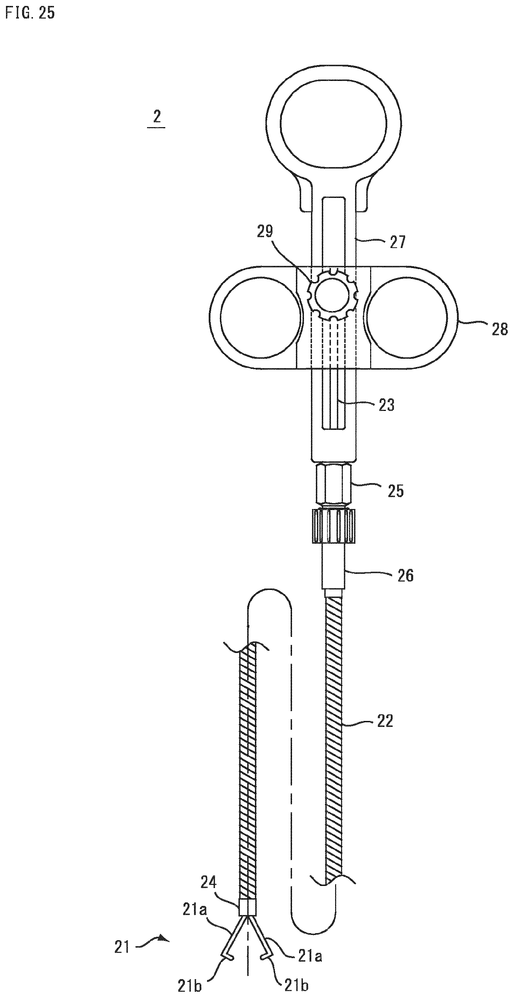

As shown in Fig.25, the ligation device 2 has a linking hook (wing member) 21, a sheath (tube) 22, a driving wire (operation wire), a locking system having a base side locking member 25 and sheath side locking member 26, an operating part having a base part 27 and slider part 28.

The sheath 22 is made of a hollow tube having flexibility, as the sheath 22, a simple tube made of resin may be used, and for the present embodiment, a coil tube is used. As the coil tube, a flat coil tube which is a long flat plate made of metal or so wound in a spiral form can be used. Note that, a round coil tube or a flat inner surface coil tube may be used. Also, as the sheath 22, a wire tube may be used. The wire tube is a tube constituted of a hollow stranded wire made of plurality of wires (cable) of metal (stainless steel) or so twisted in a spiral to form a hollow at the inside.

At a distal end of the coil tube of the sheath 22, a tip member 24 of an approximately cylinder form is integrally fixed, and a distal end face of the tip member 24 is a contact part capable of contacting with the tube 12 of the ligation tool 1A. Note that, from the point of the cost and reducing the number of parts or so, the tip member 24 may not be provided, and in this case the distal end face of the coil tube is the contact part contacting with the tube 12 of the ligation tool 1A.

The driving wire 23 is made of the wire having flexibility, and a wire rope is used in the present embodiment. The wire rope is a rope constituted by a twisted wire formed by plurality of wires (cable) of metals (stainless steel) or so twisted in a spiral form. Note that, as the driving wire 23, a wire made of single wire may be used.

At the tip (distal end) of the driving wire 23, the linking hook 21 is integrally installed. The linking hook 21 is constituted by an elastic member having a pair of arm parts 21a and 21a arranged to open in inverted V shape along the tip thereof, and a pair of claw parts 21b and 21b made by bending the tip part of the arms 21a and 21a inwards. A base end part of the arm parts 21a and 21a is integrally formed with each other, and also integrally fixed to the tip (distal end) of the driving wire 23 by welding or so.

The driving wire 23 is slid by pushing towards the distal end side with respect to the sheath 22 (that is, the sheath 22 is slid to the proximal end side with respect to the driving wire 23), thereby the linking hook 21 projects out from the distal end of the sheath 22 and opens in an inverted V shape by its own elasticity. On the other hand, the driving wire 23 is slid so to pull in from the distal end side with respect to the sheath 22 (that is, the sheath 22 is slid to the distal end side with respect to the driving wire 23), thereby the linking hook 21 is closed by placed inside of the distal end of the sheath 22.

By holding the driving wire 23 at a certain position and pushing the sheath 22, the pair of arm parts 21a and 21a of the linking hook 21 can be opened and closed (holding or releasing the holding) freely while maintaining the linking hook 21 at a certain position. As the linking hook 21, metals such as stainless steel or so can be used.

The proximal end (base end part) of the sheath 22 inserted with the driving wire 23 is connected and fixed to the distal end of the base part 27 via a lure locking system having the base side locking member 25 and the sheath side locking member 26.

At the base part 27, the slider part 28 is installed in a slidable manner. The proximal end of the driving wire 23 reaches to the slider part 28 by going through each holes of the sheath side locking member 26 and the base side locking member 25, and it is fixed in a releasable manner to the slider part 28 via a locking screw 29.

By sliding the slider part 28 to the distal end side with respect to the base part 27, the linking hook 21 provided at the distal end of the driving wire 23 is pushed out from the distal end of the sheath 22, and opens in an inverted V shape by its own elasticity. On the contrary to this, by sliding the slider part 28 to the proximal end side with respect to the base part 27, the linking hook 21 provided at the distal end of the driving wire 23 is housed to the inside of the sheath 22 by placed inside from the distal end of the sheath 22.

### (Suturing device)

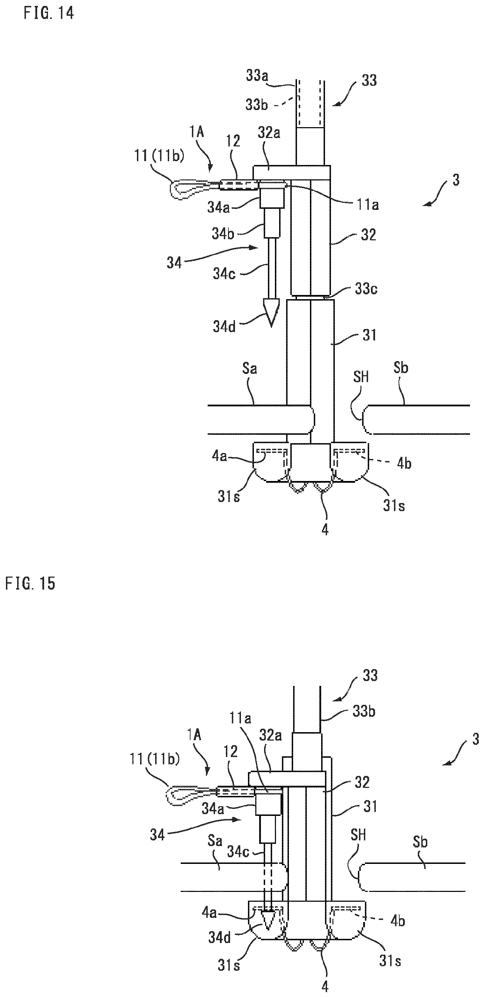

As shown in Fig.14, the suturing device 3 has a pair of front arm 31 and back arm 32, and an arm moving means 33 to move this pair of front arm 31 and back arm 32. This suturing device 3 installs the arm moving means 33 along a shaft and to an outer side of the endoscope not shown in the figure; thereby it is used by fixing to the endoscope. By verifying the movement of the pair of front arm 31 and back arm 32 by a camera of the endoscope not shown in the figure, the incision formed to the digestive tract such as stomach or so can be sutured using the pair of front arm 31 and back arm 32.

The arm moving means 33 is a long member extending along an axial direction, and is constituted by three tubes (or by two tubes and wire) having flexibility which can bend along with the bending of the shaft of the endoscope not shown in the figure. That is, the arm moving means 33 is constituted by a case tube 33a, a back arm moving tube 33b, and a front arm moving tube 33c (or a front arm moving wire).

The case tube 33a is a hollow tube form member fixed to the shaft of the endoscope not shown in the figure using an installing tool not shown in the figure. This case tube 33a is not particularly limited, and for example it can be formed by a material such as polyethylene and vinyl chloride or so.

The back arm moving tube 33b is a hollow tube form member inserted in the case tube 33a, and it is provided so that it can move along the axis of the case tube and also capable of rotating around the axis in the case tube 33a. At the tip of the back arm moving tube 33b, the back arm 32 is connected. The material of the back arm moving tube 33b is not particularly limited, and it can be formed by a tube made of resin such as polyethylene and vinyl chloride or so, and a wire tube made of metal wire or so. Particularly, the back arm moving tube 33b is rotated to swing the back arm 32, thus when the back arm moving tube 33b is rotated at the handling side, preferably the back arm 32 can be swung by the same amount as the rotation of the back arm moving tube 33b. For example, the back arm moving tube 33b may be formed by wire tubes made of plurality of metal wires provided in parallel with each other and coaxially along the axis direction; thereby the function mentioned in above can be attained.

The front arm moving tube 33c is a tube inserted in the back arm moving tube 33b, and it is provided so that it can move along the axis of the back arm moving tube 33b and also can rotate around the axis in the back arm moving tube 33b. At the tip of the front arm moving tube 33c, the front arm 31 is connected. The material of the front arm moving tube 33c is not particularly limited, and preferably the material wherein 10 mm or so of the tip part having high rigidity, and the handling side from the tip part being soft but does not shrink or expand as moving along the moving direction is used. For example, those having a rod form part with high rigidity made by metal or so at 10 mm or so of the tip part and rest of the part formed by wire or so can be used as the front arm moving tube 33c. Particularly, the front arm moving tube 33c is rotated to swing the front arm 31, hence when the front arm moving tube 33c is rotated at the handling side, preferably the front arm 31 can be swung by the same amount as the rotation of the front arm moving tube 33c.

The base end of the front arm moving tube 33c and the base end of the back arm moving tube 33b which extends to operating part and operates the shaft of the endoscope not shown in the figure. Therefore, by operating the base ends of each tube 33b and 33c, the movement of the tip of each tubes 33c and 33b can be controlled (the movement along the axial direction, and the rotation around the axis).

By moving the front arm moving tube 33c and the back arm moving tube 33b at the same time, or moving either one along the axial direction, the pair of front arm 31 and back arm 32 can be approached close to each other or move away from each other. Also, by rotating the front arm moving tube 33c around its axis, the front arm 31 can be rotated around the axis of the front arm moving tube 33c, and by rotating the back arm moving tube 33b around its axis, the back arm 32 can be rotated around the axis of the back arm moving tube 33b.

Note that, the outer diameter of the arm moving means 33 (that is, the outer diameter of the case tube 33a) only needs to be a size which can insert the endoscope not shown in the figure installed to the suturing device of the present embodiment to the digestive tract (or in the over tube), and it is not particularly limited. For example, the outer diameter of the arm moving means 33 is preferably 11 to 13 mm or so, and more preferably 11 to 12 mm or so for the diameter which includes the diameter of the arm moving means 33 and the outer diameter of the shaft of the endoscope not shown in the figure.

The back arm 32 includes a prismatic member 32a formed approximate prismatic form, and at the back arm 32, the tip of the back arm moving tube 33b is connected. Hereinafter, a center axis of the back arm moving tube 33b at the connection portion between the back arm moving tube 33b and the back arm 32 is simply referred as the center axis of the tip of the back arm moving tube 33b. Note that, at the back arm 32, a through hole (not shown in the figure) penetrating through top to bottom is formed, and the center axis of the tip of the back arm moving tube 33b is arranged to have the same axis with the center axis of the through hole.

At the tip of the prismatic member 32a of the back arm 32, a needle form member 34 is provided. This needle form member 34 has a larger diameter part 34a, an intermediate diameter part 34b, a smaller diameter part 34c, and an arrowhead form part 34d. The arrowhead part 34d has the outer diameter of the base end which is larger than the outer diameter of the tip of the smaller diameter part 34c, and it is made so to form a step at the connection part between the smaller diameter part 34c.

The needle form member 34 is installed to the prismatic member 32a of the back arm 32 so that the tip of the needle member is facing to the front arm 31, and the center axis thereof is approximately parallel with the center axis of the tip of the back arm moving tube 33b.

By rotating the back arm moving tube 33b around its center axis, the needle form member 34 can be rotated around the center axis of the tip of the back arm moving tube 33b while maintaining the center axis of the needle form member 34 parallel with the center axis of the tip of the back arm moving tube 33b.

Note that, the needle form member 34 can penetrate through the object by piercing the object to be sutured, and furthermore the material, length, and axial diameter thereof are not particularly limited as long as it has length and strength which can be pulled out from the object by moving to the opposite direction of the penetration of the object. For example, depending on the suturing device 3, in case of suturing the stomach wall, the length only needs to be the length which can penetrate through the stomach wall, and the material is preferably metal from the point of the strength. For example, the length of the needle form member 34 is preferably 7 to 20 mm or so, and more preferably 7 to 10 mm or so. Also, preferably the axial diameter of the larger diameter 34a of the needle form member 34 is 1.5 to 3.0 mm or so, the axial diameter of the intermediate diameter part 34b is 1.0 to 2.0 mm or so, the axial diameter of the smaller diameter part 34c is 0.5 to 1 mm or so, and the axial diameter of the arrowhead part 34d is 0.6 to 1.5 mm or so at the maximum diameter.

At the larger diameter part 34a of the needle form member 34, the above mentioned suture ligation apparatus 1A is mounted. The suture ligation tool 1A is mounted to the larger diameter part 34a of the needle form member 34 by sliding the tube 12a to the ligation loop 11a side while the larger diameter part 34a of the needle form member 34 is penetrating through the ligation loop 11a.

At the tip part of the front arm 31, a bifurcate part 31s formed in an approximate U shape (it may be V shape, angular U shape or so), and the suture 4 is placed across this bifurcate part 31s. At both ends of the suture 4, engaging members 4a and 4b respectively formed in a circular ring form are installed. At each tip part of the bifurcate part 31s of the front arm 31, a through hole (not shown in the figure) penetrating through the front face (lower face in Fig.14) and back face (upper face in Fig.14) is formed, and at the back face side of the through hole, a housing space (not shown in the figure) to which the engaging members 4a and 4b can engage is formed. The housing space has slightly larger diameter than the through hole, and the diameter is about the size which allows to engage the engaging members 4a and 4b in a releasable manner by the inner wall of the housing space while the through hole penetrating through front and back of the engaging members 4a and 4b is approximately concentric with respect to the through hole of the bifurcate part 31s.

The arrowhead part 34d of the needle form member 34 can be inserted to the through hole of each of engaging members 4a and 4b, and if the arrowhead part 34d is completely passed through the though hole, then the engaging members 4a and 4d will not be released from the needle form member 34. Specifically, each of engaging members 4a and 4b are formed to have smaller inner diameter than the outer diameter of the arrowhead part 34d of the needle form member 34, but is larger than the axial diameter of the tip (that is, the connection part between the arrowhead part 34d) of the smaller diameter part 34c of the needle form member 34. Note that, at the center part of the bifurcate part 31s, a suture housing part (not shown in the figure) having a depressed shaped which opens to the front face (lower face of Fig. 14) is provided, and the middle portion of the suture 4 arranged across the bifurcate part 31s is housed to this suture housing part.

### (Suturing of the incision)

Hereinafter, the suture procedure of the incision using the above mentioned suture ligation tool 1A, the ligation device 2, the suturing device 3, and the suture 4 will be explained by referring to Fig.14 to Fig.24. Note that, in below, the example of suturing the incision formed at the stomach wall will be explained.

First, as shown in Fig.14, the shaft of the endoscope not shown in the figure installed with the suturing device 3 is inserted to the stomach, then it is placed near the incision SH which is to be sutured. While in this condition, the front arm moving tube 33c of the arm moving means 33 is operated, and only the front arm 31 is inserted to the incision SH.

Then, the back arm moving tube 33b and the front arm moving tube 33c of the arm moving means 33 are operated, then the front arm 31 and the back arm 32 are arranged so that at least one of rim Sa of the incision SH is placed between either one of the bifurcate part 31s of the front arm 31 and the needle form member 34.

Then, as shown in Fig.15, the back arm moving tube 33b of the arm moving means 33 is operated, and the back arm 32 is approached close to the front arm 31, then the needle form member 34 pierces and penetrates through one of the rim Sa. Further, the arrowhead part 34d of the needle form member 34 penetrates through the through hole of the engaging member 4a housed (supported) in the housing space of one of the bifurcate part 31s. Thereby, the through hole of the engaging member 4a reaches to the smaller diameter part 34c of the needle form member 34, and the engaging member 4a is engaged to the needle form member 34.

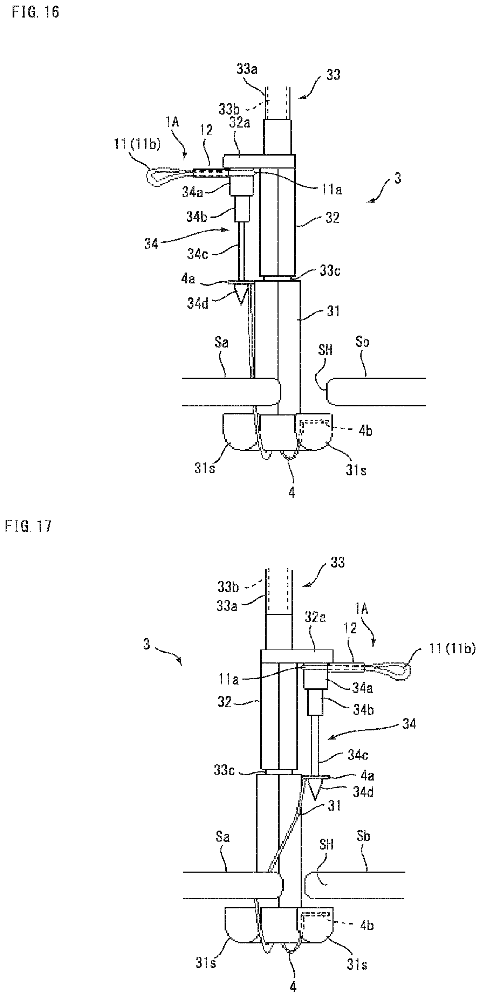

Next, as shown in Fig.16, the back arm moving tube 33b of the arm moving means 33 is operated to move the back arm 32 away from the front arm 31, then the needle form member 34 of which the engaging member 4a is engaged returns to the inside of the stomach by moving backwards of the hole (herein after it will be referred as a first pierced hole) formed when the needle form member 34 is pierced through one of the rims Sa. Thereby, part of the suture 4 (part at the engaging member 4a side) is penetrated through one of the rim Sa.

Then, as shown in Fig.17, the front arm 31 and the back arm 32 are arranged so that other rim Sb of the incision SH is placed between other bifurcate part 31s of the front arm 31 and the needle form member 34.

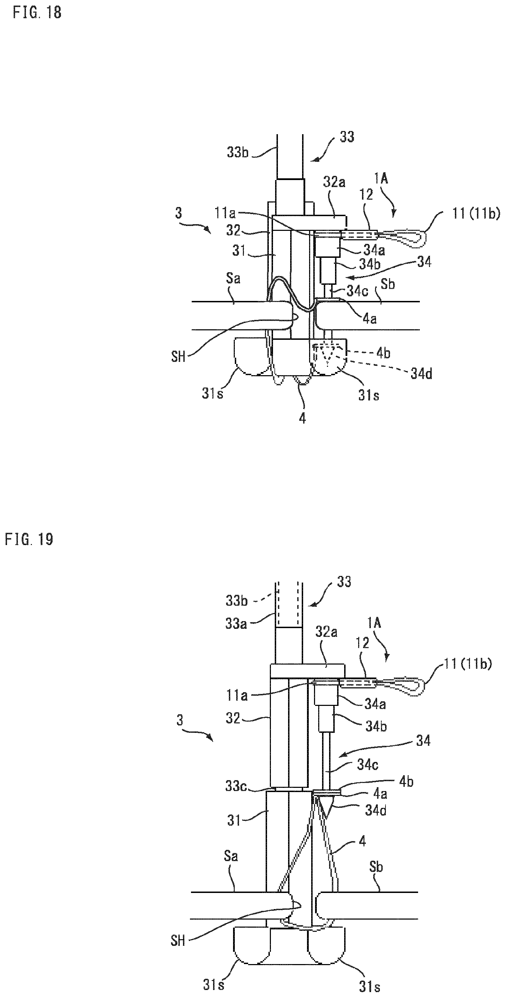

Then, as shown in Fig.18, the back arm moving tube 33b of the arm moving means 33 is operated to move the back arm 32 closer to the front arm 31, then the needle form member 34 pierces and penetrates through the other rim Sb. Further, the arrowhead part 34d of the needle form member 34 penetrates through the through hole of the engaging member 4b housed (supported) in the housing space of other bifurcate part 31s. Thereby, the through hole of the engaging member 4b reaches to the smaller diameter part 34c of the needle form member 34, and the engaging member 4b is engaged to the needle form member 34.

Next, as shown in Fig.19, the back arm moving tube 33b of the arm moving means 33 is operated to move the back arm 32 away from the front arm 31, then the needle form member 34 of which the engaging member 4b is engaged returns to the inside of the stomach by moving backwards the hole (herein after it will be referred as a second pierced hole) formed when the needle form member 34 is pierced through other rim Sb. Thereby, part of the suture 4 (part at the engaging member 4b side) is penetrated through other rim Sb.

Thereby, the pair of engaging members 4a and 4b which the both ends of the suture 4 are fixed will be engaged to one needle form member 34, thus the suture 4 forms a ring which goes out of the stomach by penetrating through the first piercing hole from the needle form member 34 (that is, inside of the stomach), then returns to the needle form member 34 (that is, inside of the stomach) by penetrating through the second pierced hole from the outside face of the stomach.

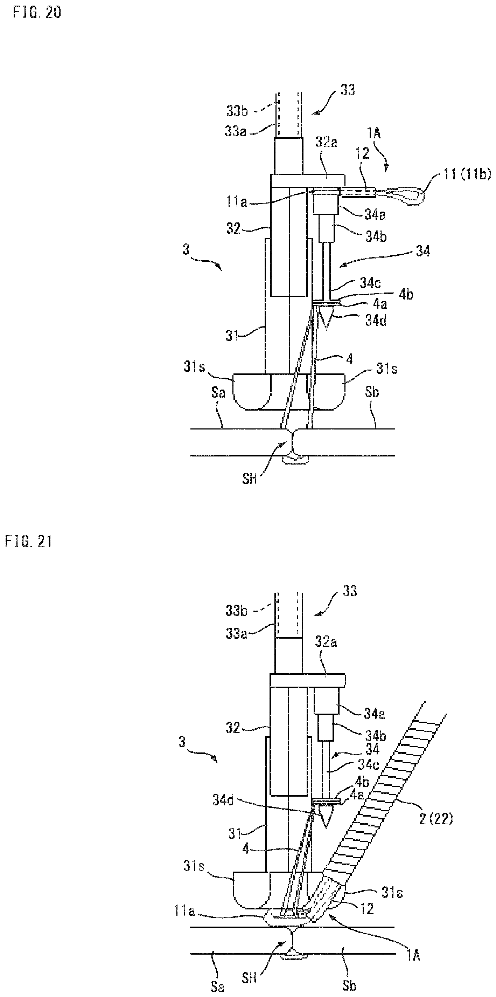

Next, as shown in Fig.20, by operating the arm moving means 33, the front arm 31 (bifurcate part 31s) is moved to the inside of the stomach through the incision SH. When the front arm 31 (bifurcate part 31s) enters to the inside of the stomach, the needle form member 34 moves away from the incision SH, and the both ends of the suture 4 move away from the incision SH, thus part of the suture 4 which penetrated through the first pierced hole and the second pierced hole are pulled toward each other, and the end faces of the pair of rims Sa and Sb of the incision SH are joined by contacting with each other.

Once the suturing procedure by the suturing device 3 of the incision SH is completed, next the ligation procedure of the suture 4 is carried out. For this procedure, the ligation device 2 shown in Fig.25 is used. First, the sheath 22 of the above mentioned ligation device 2 is inserted via an endotherapy accessory guide pipe of the endoscope, and the distal end of the sheath 22 is positioned near the linking loop 11b of the ligation tool 1A installed to the larger diameter part 34a of the needle form member 34. Note that, this insertion procedure is carried out while the linking hook 21 provided at the distal end of the driving wire 23 is housed inside the sheath 22 by placed in from the distal end of the sheath 22 by sliding the slider part 28 to the proximal end side with respect to the base part 26.

Then, the slider part 28 is slid to distal end side with respect to the base part 27, and the linking hook 21 provided at the distal end of the driving wire 23 is pushed out (project out) from the sheath 22, then the pair of arms parts 21a and 21a opens in an inverted V shape by its own elasticity.

Next, the linking loop 11b of the ligation tool 1A is made closer so that the pair of arm parts 21a and 21a of the linking hook 21 can hold it. Under this condition, the position of the base part 27 is slid to the distal end side with respect to the slider part 28 while keeping the position of the slider part 28, then the sheath 22 moves to the distal end side, and the pair of arm parts 21a and 21a projecting out from the distal end of the sheath 22 are pulled into the sheath 22 and the pair of arm parts 21a and 21a are closed.

Thereby, the linking loop 11b is held by the pair of arm parts 21a and 21a (the claw parts 21b and 21b) of the linking hook 21 without changing the position of the ligation tool 1A. Further, under this condition, by sliding the base part 27 to the distal end side with respect to the slider part 28, the linking loop 11b held by the pair of arm parts 21a and 21a is pulled in from the distal end of the sheath 22, and the distal end face of the sheath 22 contacts with the end face of the tube 12. Thereby, while the ligation tool 1A has not ligated, the distal end of the sheath 22 of the ligation device 2 is connected with the ligation tool 1A.

Next, as shown in Fig.21, the tip part of the sheath 22 connected with the ligation tool 1A is moved towards the incision SH side (downwards in Fig.21), and the both ends of the suture 4 are passed through the inside of the ligation loop 11a of the ligation tool 1A. Thereby, the suture 4 is tightened, and under this condition, the base part 27 is further slid to the distal end side with respect to the slider part 28, then the linking loop 11b is pulled inside of the sheath 22, then the tube 12 is slid to the ligation loop 11a side by the distal end face of the sheath 22, and the both ends of the suture 4 are tied up.

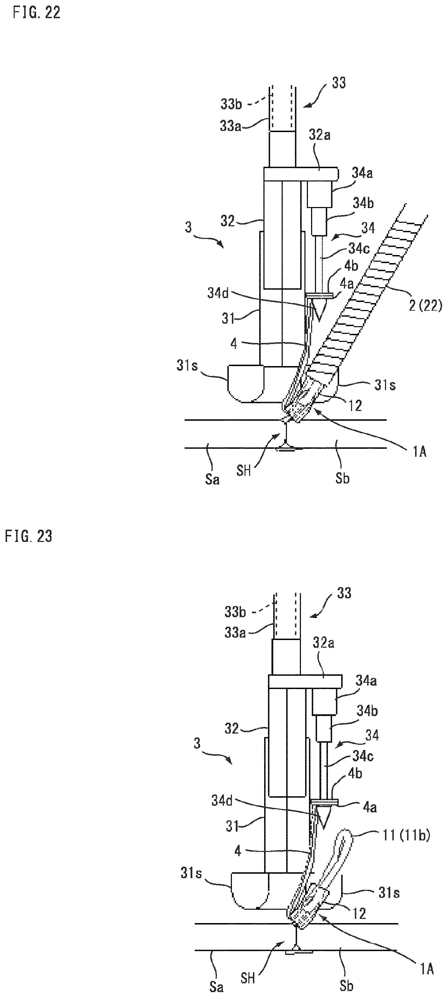

While the both end parts of the suture 4 are tied up, the base part 27 is further slid to the distal end side with respect to the slider part 28, then as shown in Fig.22, the ligation loop 11a is pulled into the tube 12 together with the suture 4, thereby the ligation loop 11a of the main body part 11 and the suture 4 are housed into the tube 12 while in close contact and compressed with each other. That is, the main body part 11 and the suture 4 are housed in the tube 12 by interference fit, thus the suture 4 and the main body part 11 are fixed without slipping out of the tube 12. Thereby, the ligation of the suture 4 is completed.

Once the ligation of the suture 4 is completed, the base part 27 is slid to the proximal end side with respect to the slider part 28 while keeping the position of the slider part 28, then the sheath 22 moves to the proximal end side, and the linking hook 21 and the linking loop 11b project out (expose) from the distal end of the sheath 22. Then, the pair of arm parts 21a and 21a of the linking hook 21 opens by its own elasticity, thus the linking loop 11b is released from being held, and as shown in Fig.23, the ligation tool 1A which has ligated is separated from the ligation device 2.

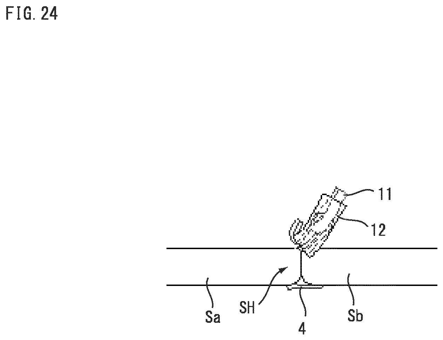

Lastly, as shown in Fig.24, the suture 4 between the ligation tool 1A and the suturing device 3 is cut by endoscopic scissor forceps called a loop cutter to separate the ligation tool 1A and the suturing device 3, and further if needed, the linking loop 11b of the ligation tool 1A which is ligated can be cut by the loop cutter or so and collected, thereby series of procedures are completed.

The first embodiment has the caulking member 13 which is fitted to the tube 12 so that part of the lumen of the tube 12 is narrow, this caulking member 13 is the locking means which locks the tip part (turning point) of the ligation loop 11a to the inside of the tube 12 so that it does not slip out of the tube. Thereby, this prevents the main body part 11 from being released from the tube 12. Thus, the ligation of the suture 4 can be carried out in good condition. The main body part 11 is released from the tube 12 when the ligation loop 11a is pulled too much with respect to the tube 12 in order to tightly ligate the suture 4 causing the ligation loop 11a to slip out of the tube 12, and releasing the main body part 11 from the tube 12. Note that, as in case of the ligation tool 1A of the first embodiment, the ligation tool 1 has the locking means which locks part of the ligation loop 11a to the inside of the tube and when the entire ligation loop 11a is pulled to the tube 12 side so that the ligation loop 11a is not left at the outside of the tube 12 where the ligation loop 11a was present, thereby the extra suture during the ligation procedure is pulled into the tube 12, and the suture 4 can be easily ligated tightly.

### [Second embodiment]

Fig.3 is a perspective view showing the constitution of the endoscopic suture ligation tool according to the second embodiment of the present invention, and Fig.4 is a cross section showing the endoscopic suture ligation tool according to the second embodiment when ligating, and also the cross section around the endoscopic suture ligation tool. Note that, the same numbers are given for the constitution parts same as the above mentioned first embodiment, and part of or entire descriptions of these are omitted.

The endoscopic suture ligation tool 1B of the second embodiment has a stopper 14 made of resin or so as the locking means to lock the tip part (turning point) of the ligation loop 11a to the inside of the tube 12 so that it does not slip out of the lumen of the tube 12 when ligating the suture 4.

The stopper 14 has a circular plate part (plate part) having larger diameter than the inner diameter of the tube 12, and the projection part 14b integrally formed to the circular plate part 14a at approximately center part of the circular plate part 14a and projecting out to the tube 12 side so that the projection part 14b can be inserted to the lumen of the tube 12. At the circular plate part 14a, a through hole 14c where part of the linking loop 11b penetrates through in a slidable manner, and a through hole 14d where other part of the linking loop 11b penetrates through in a slidable manner are formed.

Although it is not shown in the figure, in order to arrange the linking loop 11b of the main body part (loop member) 11 by penetrating through each of the through holes 14c and 14d of the circular plate part 14a, at the circular plate part 14a, a pair of split grooves which respectively reach to the through holes 14c and 14d from the outer face are formed. By pushing and opening this split grooves, the linking loop 11b can be fit. Note that, instead of forming such split grooves, the wire member constituting the main body part 11 can be passed through each of the through holes 14c and 14d of the circular plate part 14a, then it may be bound in a loop form.

The outer diameter of the circular plate part 14a is 4.0 mm in the present embodiment. The thickness of the circular plate 14a is 0.5 mm in the present embodiment. The inner diameter of the through holes 14c and 14d is slightly larger than the wire diameter of the main body part 11, and it is 0.5 mm in the present embodiment. The length (height) of the projection part 14b is 2.0 mm in the present embodiment. The outer diameter of the projection part 14b is 1.5 mm in the present embodiment.

During ligation, while holding the linking loop 11b of the ligation tool 1B with the linking hook 21 of the ligation device 2, the circular plate part 14a is placed between the ligation device 2 and the tube 12; thereby the distal end of the sheath 22 of the ligation device 2 is contacted with the circular plate part 14a. When the ligation loop 11a is pulled into the tube 12 together with the suture 4 by sliding the tube 12 to the ligation loop 11a side, the suture 4 which is passed through the inside of the turning point (tip portion) of the ligation loop 11a or through this part contacts with the tip of the projection part 14b and locked. Thereby, this securely prevents the ligation loop 11a from slipping out the tube 12, which is caused by pulling the ligation loop 11a (main body part 11) too much with respect to the tube 12 in order to tightly ligate the suture 4 causing the ligation loop 11a to slip out of the tube 12.

### [Third embodiment]

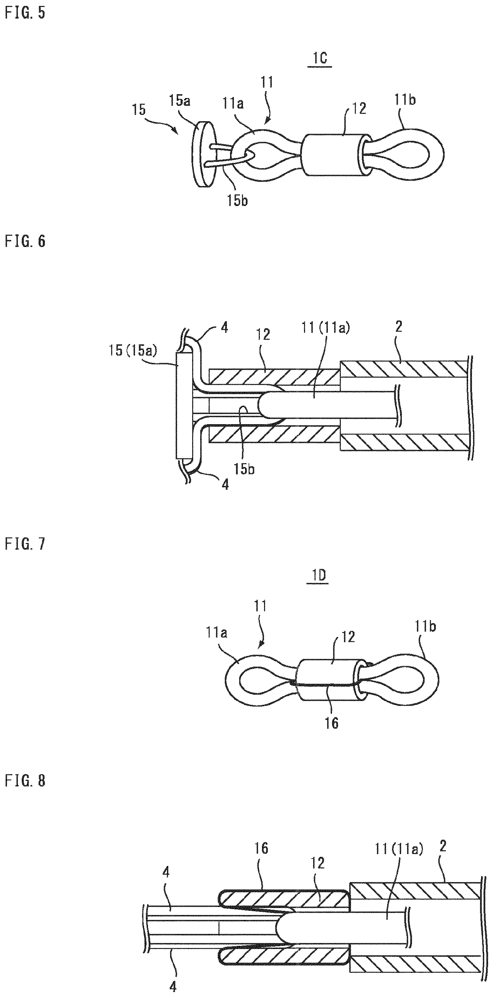

Fig.5 is a perspective view showing the constitution of the endoscopic suture ligation tool according to the third embodiment of the present invention, and Fig.6 is a cross section of the endoscopic suture ligation tool according to the third embodiment during the ligation, and also the cross section around the endoscopic suture ligation tool. Note that, the same numbers are given for the constitution parts same as the above mentioned first embodiment, and part or entire descriptions of these are omitted.

The endoscopic suture ligation tool 1C of the third embodiment has a stopper 15 as the locking means to lock the tip part (turning point) of the ligation loop 11a to the inside of the tube 12 so that it does not slip out of the lumen of the tube 12 when ligating the suture 4.

The stopper 15 has a circular plate part (plate part) 15a having larger diameter than the inner diameter of the tube 12, and a connection loop 15b having approximate U shape. One end of the connection loop 15b is integrally connected to part (the part slightly off-centered from the center part) of the circular plate part 15b, and the other end is integrally connected to other part (the part slightly off-centered from the center part, and 180 degrees opposite to the one end) of the circular plate part 15a.

The outer diameter of the circular plate part 15a is 4.00 mm in the present embodiment. The thickness of the circular plate part 15a is 0.50 mm in the present embodiment. The length (height) of the connection loop 15b is 1.75 mm in the present embodiment. The outer width of the connection loop 15b is 1.5 mm in the present embodiment. The inner width of the connection loop 15b is 0.50 mm in the present embodiment. Also, the material of the stopper 15 is not particularly limited as long as at least the circular plate part 15a is formed by the material having appropriate rigidity, and in the present embodiment, the entire stopper 15 is formed by stainless steel.

In case of ligating using the ligation device 2, when the ligation loop 11a is pulled into the tube 12 together with the suture 4 by sliding the tube 12 to the ligation loop 11a side, the connection loop 15b of the stopper 15 enters into the tube 12, and the circular plate part 15a interferes the end face of the tube 12, thereby it is locked. Thereby, this securely prevents the ligation loop 11a from slipping out the tube 12, which is caused by pulling the ligation loop 11a too much with respect to the tube 12 in order to tightly ligate the suture 4 causing the ligation loop 11a to slip out of the tube 12.

### [Fourth embodiment]

Fig.7 is a perspective view showing the constitution of the endoscopic suture ligation tool according to the fourth embodiment of the present invention, and Fig.8 is a cross section of the endoscopic suture ligation tool according to the fourth embodiment during the ligation, and also the cross section around the endoscopic suture ligation tool. Note that, the same numbers are given for the constitution parts same as the above mentioned first embodiment, and part or entire descriptions of these are omitted.

The endoscopic suture ligation tool 1D of the fourth embodiment has a stopper ring 16 as the locking means to lock the tip part (turning point) of the ligation loop 11a to the inside of the tube 12 so that it does not slip out of the lumen of the tube 12 when ligating the suture 4.

The stopper ring 16 is made from the wire member (string) having a loop form (endless form), and it is a member made of endless wire member wound around the tube 12, wherein the stopper ring passes through the linking loop 11b and runs along one side face of the tube 12, and passes through the ligation loop 11a and runs along the other side face of the tube 12.

As the material of the stopper ring 16, for example nylon can be used. The diameter of wire constituting the stopper ring 16 is 0.3 mm in the present embodiment. The length (circumference) of the stopper ring 16 is 15 mm in the present embodiment.

In case of ligating using the ligation device 2, when the ligation loop 11a is pulled into the tube 12 together with the suture 4 by sliding the tube 12 to the ligation loop 11a side, part of the stopper ring 16 is also pulled into the tube 12 with these, and will be locked due to a tension of the stopper ring at the position defined by the relation between the length (circumference) of the stopper ring 16. Thereby, this securely prevents the ligation loop 11a from slipping out of the tube 12, which is caused by pulling the ligation loop 11a too much with respect to the tube 12 in order to tightly ligate the suture 4 causing the ligation loop 11a to slip out of the tube 12.

### [Fifth embodiment]

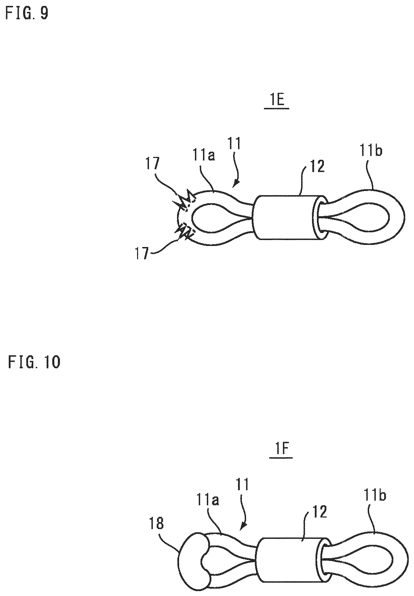

Fig.9 is a perspective view showing the constitution of the endoscopic suture ligation tool according to the fifth embodiment of the present invention. Note that, the same numbers are given for the constitution parts same as the above mentioned first embodiment, and part or entire descriptions of these are omitted.

The endoscopic suture ligation tool 1E of the fifth embodiment has plurality of projection parts 17 formed integrally with the ligation loop 11a of the main body part (loop member) 11 as the locking means to lock the tip part (turning point) of the ligation loop 11a to the inside of the tube 12 so that it does not slip out of the lumen of the tube 12 when ligating the suture 4.

As the position to provide the projection parts 17, the area around the turning point (tip portion) of the ligation loop 11a is preferable. The height of the projection parts 17 is 0.2 mm in the present embodiment. The number of projection parts 17 is not particularly limited, and it may be 1 or more.

Compared to the case not having the projection parts, the resistance against the inner face of the tube 12 is larger when the ligation loop 11a is pulled into the tube 12 together with the suture 4 by sliding the tube 12 to the ligation loop 11a side during the ligation due to the projection parts 17 formed integrally with the ligation loop 11a. Thereby, the ligation loop 11a is suppressed from slipping out of the tube 12, which is caused by pulling the ligation loop 11a too much with respect to the tube 12. Note that, if the ligation loop 11a is pulled strongly into the tube 12 together with the suture 4 during the ligation, there is a chance that the ligation loop 11a slipping out of the tube 12, hence for example it is effective to use with the caulking member 13 of the first embodiment as these will act synergistically.

### [Sixth embodiment]

Fig.10 is a perspective view showing the constitution of the endoscopic suture ligation tool according to the sixth embodiment of the present invention. Note that, the same numbers are given for the constitution parts same as the above mentioned first embodiment, and part or entire descriptions of these are omitted.

The endoscopic suture ligation tool 1F of the sixth embodiment has a larger diameter part 18 having larger diameter than other parts of the ligation loop 11a which is integrally formed to the ligation loop 11a of the main body part 11 as the locking means to lock the tip part (turning point) of the ligation loop 11a to the inside of the tube 12 so that it does not slip out of the lumen of the tube 12 when ligating the suture 4.

As the position to provide the larger diameter part 18, the turning point (tip portion) of the ligation loop 11a and the area around there are preferable. The outer diameter of the larger diameter part 18 is 0.6 mm in the present embodiment.

Compared to the case not having the larger diameter part, the resistance against the inner face of the tube 12 is larger when the ligation loop 11a is pulled into the tube 12 together with the suture 4 by sliding the tube 12 to the ligation loop 11a side during the ligation due to the larger diameter part 18 formed integrally with the ligation loop 11a. Thereby, the ligation loop 11a is suppressed from slipping out of the tube 12, which is caused by pulling the ligation loop 11a too much with respect to the tube 12. Note that, if the ligation loop 11a is pulled strongly into the tube 12 together with the suture 4 during the ligation, there is a chance that the ligation loop 11a slipping out of the tube 12, hence for example it is effective to use with the caulking member 13 of the first embodiment as these will act synergistically.

### [Seventh embodiment]

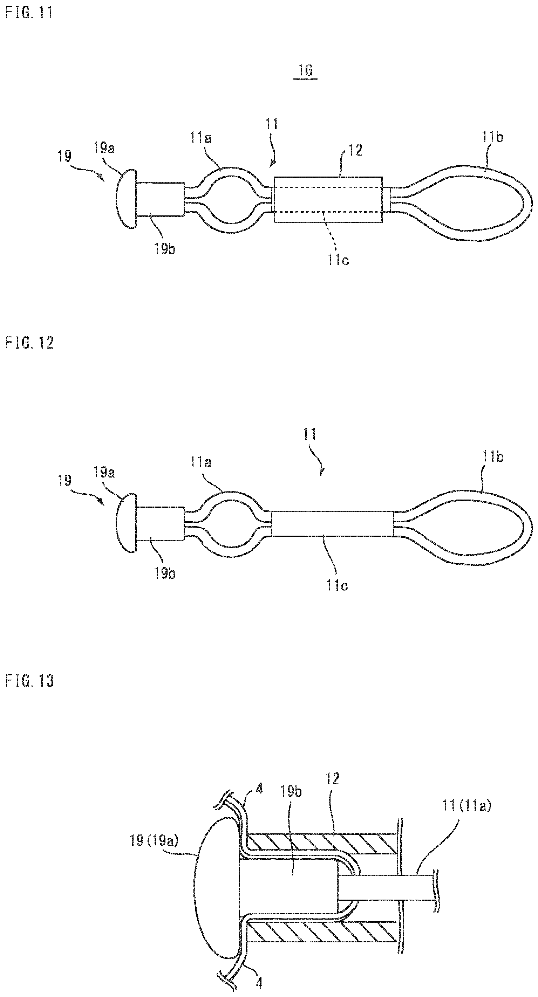

Fig.11 is a plan view showing the constitution of the endoscopic suture ligation tool according to the embodiment of the present invention, Fig.12 is the plan view showing the condition prior to fitting the tube of the endoscopic suture ligation tool, and Fig.13 is a partial cross section showing the ligation of the suture by the endoscopic suture ligation tool. Note that, the same numbers are given for the constitution parts same as the above mentioned first embodiment, and part or entire descriptions of these are omitted.

The endoscopic suture ligation device 1G has the main body part 11 and the tube 12. The main body part 11 has the ligation loop 11a for passing through the suture 4 which will be described in below, the linking loop 11b connected with the ligation device 2 which will be described in below, and the connecting part 11c connecting the ligation loop 11a and the linking loop 11b. The connecting part 11c is a columnar part (a circular column in the present embodiment) wherein the ligation loop 11a is integrally connected to one end of the connecting part 11c and the linking loop 11b is integrally connected to other end of the connecting part 11c.

The tube 12 is fit to the connecting part 11c in a slidable manner. The tube 12 is a member (tightening tube) having approximately cylinder form made of a material having elasticity, and the ligation loop 11a is pulled into the lumen of the tube 12 by sliding it to the ligation loop 11a side from the connecting part 11c.

The ligation loop 11a has a locking part 19 (19a and 19b) as the locking means for locking part of the ligation loop 11a so that the ligation loop does not slip out of the tube 12 by pulling too much into the tube 12.

The material of the ligation loop 11a and the linking loop 11b is not particularly limited as long as it has appropriate flexibility, and for example polyamide resin, polypropylene resin, styrene based elastomer or so can be used. In the present embodiment, the entire main body part 11 which includes the ligation loop 11a, the linking loop 11b, the connecting part 11c, and the locking part 19 is constituted by same material. Also, the material of the tube 12 is not particularly limited as long as it has appropriate elasticity, and for example silicone elastomer can be used.

While the suture 4 of which the both ends are tied up is passed through the ligation loop 11a, the tube 12 is slid to the ligation loop 11a side (or the linking loop 11b is pulled with respect to the tube 12) so that the ligation loop 11a is pulled into the tube 12 using the ligation device 2. Thereby, as shown in Fig. 13, the neck part 19b of the locking part 19 and the ligation loop 11a are pulled into the tube 12 together with the suture 4, and the end portion (the portion where the locking part 19 is provided) which is opposite side of the connecting part 11c of the ligation loop 11a is pulled into the middle portion of the tube 12, and the suture 4 is ligated.

When the ligation loop 11a is pulled into the tube 12 together with the suture 4, the tube 12 expands outwards due to the pressure from the neck part 19b of the locking part 19 and the suture 4, and by the restoring force (tightening force) of the tube 12, the suture 4, the ligation loop 11a, and the neck part 19b of the locking part 19 are firmly held in the tube 12.

The diameter of the wire member constituting the ligation loop 11a and the linking loop 11b can be for example within the range of 0.3 to 0.8 mm. The size in the axial direction of the connecting part 11c can be for example within the range of 3 to 20 mm. The size in the axial direction of the tube 12 is for example within the range of 3 to 10 mm. The outer diameter of the tube 12 can be for example within the range of 1 to 2 mm. The inner diameter of the tube 12 can be for example within the range of 0.1 to 0.8 mm.

The present embodiment has the locking part 19 as the locking means to lock the ligation loop 11a in the tube 12 so that the tip part (the opposite end part of the connecting part 11c) of the ligation loop 11a does not pass through and slip out of the lumen of the tube 12.

The locking part 19 has the head part 19a and the neck part 19b. The neck part 19b is a part having an approximately circular columnar form, and has a diameter which can be inserted or pressed into the lumen of the tube 12 (the diameter same as the lumen of the tube 12 or slightly larger). The head part 19a is a part having approximate circular plate form, and has a diameter which cannot be inserted or pressed into the lumen of tube 12 (larger diameter than the diameter of the neck part 19b). Note that, the head part 19a is not limited to the approximate circular plate form, and it may be other form. The head part 19a may have part of or entire size larger than the inner diameter of the tube 12 so that it cannot be inserted or pressed into the lumen of the tube 12.

The outer diameter of the head part 19a can be for example within the range of 0.8 to 2.0 mm. The length (size) in the axial direction of the neck part 19b can be for example within the range of 1 to 5 mm. The diameter of the head part 19b can be for example within the range of 0.6 to 1.6 mm.

Part of or all of the ligation loop 11a, the linking loop 11b, the connecting part 11c, and the locking part 19 (the head part 19a, the neck part 19b) constituting the main body part 11 can be formed separately and then connected and fixed so that these are integral with each other. However, from the point of the easiness of the production and low cost or so, preferably the entire parts thereof may be integrally formed at the same time by an injection molding using the same materials.

During the ligation using the ligation device 2, the tube 12 is slid to the ligation loop 11a side, and the ligation loop 11a is pulled into the tube 12 together with the suture 4, then the neck part 19b of the locking part 19 enters into the tube 12, and the head part 19a contacts with the end face of the tube 12 thereby it is locked. Thereby, this prevents the ligation loop 11a from slipping out of the tube 12, which is caused by pulling the ligation loop 11a too much with respect to the tube 12 in order to tightly ligate the suture 4 causing the ligation loop 11a to slip out of the tube 12.

Note that, in the present embodiment, the connecting part 11c is provided as the circular columnar part or member, and at one end of the connecting part 11c is provided with the ligation loop 11a at one end, and the linking loop 11b is provided at the other end, but it is not limited thereto. For example, as discussed in above mentioned first to sixth embodiments, the tube 12 is fit at the middle part of the loop member made of flexible wire having endless form so that the loop member inside the tube is facing against each other, and one side of the loop formed at one side of the tube 12 may be the ligation loop 11a, and the loop formed at the other side of the tube 12 may be linking loop 11b, and the part where the tube 12 is fit may be the connecting part 11c.

Note that, if the tube 12 is fitted to the middle part of the loop member where the loop member faces against each other, the form (shape) of the ligation loop 11a or the linking loop 11b is unstable, and sufficient loop form (circular form or close to this form having enough space at the inside) may not be maintained for installing the ligation tool 1G to the needle form member 34 (see Fig.14) of the suturing device 3 mentioned in the above, or for holding and linking the linking loop 11b to the ligation device 2, thus in some case it was difficult to carry out the installing procedure and linking procedure.

Compared to this, the constitution of the seventh embodiment (the constitution of providing the connecting part 11c as the member or part having an approximate circular columnar form, and providing the ligation loop 11a at the one end and providing the linking loop 11b at the other end), the form (shape) of the ligation loop 11a and the linking loop 11b have excellent stability, thus sufficient loop form can be secured (circular shape or close to this shape having enough space at the inside) for installing the ligation tool 1G to the needle form member 34 (see Fig.14) of the suturing device 3 mentioned in the above, or for holding and linking the linking loop 11b to the ligation device 2, thus the installing procedure and linking procedure can be carried out easily.

The above mentioned seventh embodiment is provided with the locking part 19 (the head part 19a, the neck part 19b) at the end part of the opposite side of the connecting part 11c of the ligation loop 11a as the locking means to lock the tip part (turning point) of the ligation loop 11a to the inside of the tube 12 so that it does not slip out of the lumen of the tube 12 when ligating the suture 4. Thereby, this prevents the main body part 11 from being released from the tube 12. Thus, the ligation of the suture 4 can be carried out in good condition. The main body part 11 is usually released when the ligation loop 11a is pulled too much with respect to the tube 12 in order to tightly ligate the suture 4 causing the ligation loop 11a to slip out of the tube 12, and releasing the main body part 11 from the tube 12. Note that, as in case of the ligation tool 1G of the above mentioned embodiment, the ligation tool 1G has the locking means which locks part of the ligation loop 11a to the inside of the tube 12 while the entire ligation loop 11a is pulled to the tube 12 side so that the ligation loop 11a is not left outside of the tube 12 where the ligation loop 11a was present, thereby the extra suture during the ligation procedure is pulled into the tube 12, and the suture 4 can be easily ligated tightly.

Note that the above mentioned embodiments are described in order to make the understanding of the invention easier, and the present invention is not to be limited thereto. Therefore, each element described in the above mentioned embodiments include all of designing modifications within the scope of the present invention.

### NUMERICAL REFEENCES

1A, 1B, 1C, 1D, 1E, 1F, 1G...Suture ligation tool
11...Main body part (loop member)
11a...Ligation loop
11b...Linking loop
11c...Connecting part
12...Tube
13...Caulking member (locking means)
14...Stopper (locking means)
14a...Circular end plate
14b... Projection part
14c, 14d...Through hole
15...Stopper (locking means)
15a...Circular plate part
15b...Connecting loop
16...Stopper ring (locking means)
17...Projection part (locking means)
18...Larger diameter part (locking means)
19...Locking part (locking means)
19a...Head part
19b...Neck part
2...Ligation device
21...Linking hook
21a...Arm part
21b...Claw part
22... Sheath
23...Driving wire
25...Base side locking member
26...Sheath side locking member
27...Base part
28...Slider part
3...Suturing device
31...Front arm
31s...Bifurcate part
32...Back arm
33...Arm moving means
33a...Case tube
33b...Back arm moving tube
33c...Front arm moving tube
34...Needle form member
34a...Larger diameter part
34d...Arrowhead part
4... Suture
4a, 4b...Engaging member
SH...Incision
Sa, Sb...Rim

## Claims

1. An endoscopic suture ligation tool for ligating a suture suturing a lumen wall of a gastrointestinal tract, comprising:
a ligation loop (11a) for passing a suture (4) through,
a linking loop (11b) connectable to a ligation device (2),
a connecting part (11c) connecting the ligation loop and the linking loop,
a tube (12) fitted in a slidable manner over the connecting part such that the ligation loop is on one side of the tube and the linking loop is on the opposite side of the tube, wherein the tube is configured to be slid towards the ligation loop side or the linking loop is configured to be pulled with respect to the tube so that the entire ligation loop is pulled into a lumen of the tube with the suture, and
a locking means provided to the ligation loop, the linking loop or the tube to lock a part of the ligation loop so that the ligation loop is not released from an inside of the tube when pulling the entire ligation loop into the lumen of the tube.

2. The endoscopic suture ligation tool according to claim 1, wherein the connecting part is a columnar part, and the ligation loop is connected to one end of the connecting part and the linking loop is connected to the other end of the connecting part.

3. The endoscopic suture ligation tool according to claim 1, wherein
middle parts facing each other of a loop member made of a flexible endless wire member are brought into contact and the tube is fit outside thereof so that the ligation loop is formed of the loop member at one side of the tube, and the linking loop is formed of the loop member at the other side of the tube, and
the connecting part is a part where the tube is being fit.

4. The endoscopic suture ligation tool according to any one of claims 1 to 3, wherein the locking means includes a caulking member fit to the tube so that a part of the lumen of the tube is narrower.

5. The endoscopic suture ligation tool according to any one of claims 1 to 3, wherein the locking means includes a stopper comprising,
a plate part having a first through hole where a part of the linking loop passes through in a slidable manner and a second through hole where other part of the linking loop passes through, and
a projection part formed integrally with the plate part at about a center of the plate part while projecting out to the tube side so that the projection part can be inserted to the lumen.

6. The endoscopic suture ligation tool according to claims 1 to 3, wherein the locking means includes a stopper comprising,
a plate part having a larger diameter than an inner diameter of the tube, and
a connection loop having approximately U shape and capable of being inserted into the lumen of the tube wherein one end of the connection loop is integrally connected to a part of the plate part, an intermediate part of the connection loop is passed through the ligation loop, and the other end of the connection loop is integrally connected with other part of the plate part.

7. The endoscopic suture ligation tool according to any one of claims 1 to 3, wherein the locking means includes a stopper ring made of endless wire member wound around the tube, and the stopper ring passes through the linking loop while running along a side face of one of outer sides of the tube, and the stopper ring passes through the ligation loop while running along the other of the outer sides of the tube.

8. The endoscopic suture ligation tool according to any one of claims 1 to 3, wherein the locking means includes a projection part integrally formed with the ligation loop.

9. The endoscopic suture ligation tool according to any one of claims 1 to 3, wherein the locking means includes a larger diameter part formed integrally with the ligation loop, the larger diameter part having a larger diameter than a diameter of the other part of the ligation loop.

10. The endoscopic suture ligation tool according to any one of claims 1 to 3, wherein the locking means includes a locking part having larger size than an inner diameter of the tube, and provided to an end part of the ligation loop opposite of the connecting part.

## Patentansprüche

1. Endoskopisches Nahtligaturinstrument zum Ligieren einer Naht, die eine Lumenwand eines Magen-Darm-Trakts vernäht, umfassend:
eine Ligaturschlaufe (11a) zum Durchführen eines Fadens (4),
eine Verbindungsschlaufe (11b), die mit einer Ligaturvorrichtung (2) verbindbar ist,
ein Verbindungsteil (11c), das die Ligaturschlaufe und die Verbindungsschlaufe verbindet,
ein Rohr (12), das verschiebbar über das Verbindungsteil gestülpt ist, so dass sich die Ligaturschlaufe auf einer Seite des Rohrs und die Verbindungsschlaufe auf der gegenüberliegenden Seite des Rohrs befindet, wobei das Rohr so ausgebildet ist, dass es in Richtung der Ligaturschlaufenseite verschoben werden kann, oder die Verbindungsschlaufe so ausgebildet ist, dass sie relativ zum Rohr gezogen werden kann, so dass die gesamte Ligaturschlaufe zusammen mit dem Nahtmaterial in ein Lumen des Rohrs gezogen wird, und
eine Verriegelungseinrichtung, die an der Ligaturschlaufe, der Verbindungsschlaufe oder dem Rohr vorgesehen ist, um einen Teil der Ligaturschlaufe zu verriegeln, so dass die Ligaturschlaufe beim Einziehen der gesamten Ligaturschlaufe in das Lumen des Rohrs nicht aus dem Inneren des Rohrs freigegeben wird.

2. Endoskopisches Nahtligaturinstrument nach Anspruch 1, wobei das Verbindungsteil ein säulenförmiger Abschnitt ist und die Ligaturschlaufe mit einem Ende des Verbindungsteils verbunden ist und die Verbindungsschlaufe mit dem anderen Ende des Verbindungsteils verbunden ist.

3. Endoskopisches Nahtligaturinstrument nach Anspruch 1, wobei
einander gegenüberliegende Mittelteile eines Schlaufenelements aus einem flexiblen endlosen Drahtelement in Kontakt gebracht werden und das Rohr außen daran angebracht wird, so dass die Ligaturschlaufe aus dem Schlaufenelement auf einer Seite des Rohrs gebildet wird und die Verbindungsschlaufe aus dem Schlaufenelement auf der anderen Seite des Rohrs gebildet wird, und
das Verbindungsteil ein Abschnitt ist, an dem das Rohr aufgesetzt ist.

4. Endoskopisches Nahtligaturinstrument nach einem der Ansprüche 1 bis 3, wobei die Verriegelungseinrichtung ein an den Schlauch angepasstes Verengungselement umfasst, so dass ein Teil des Lumen des Schlauchs verengt ist.

5. Endoskopisches Nahtligaturinstrument nach einem der Ansprüche 1 bis 3, wobei die Verriegelungseinrichtung einen Stopper umfasst, der Folgendes umfasst:
einen Plattenteil mit einem ersten Durchgangsloch, durch das ein Teil der Verbindungsschlaufe gleitend hindurchgeführt wird, und einem zweiten Durchgangsloch, durch das ein anderer Teil der Verbindungsschlaufe hindurchgeführt wird, und
ein Vorsprungsteil, das einst ü ckig mit dem Plattenteil etwa in dessen Mitte ausgebildet ist und zur Rohrseite hin vorsteht, sodass das Vorsprungsteil in das Lumen eingeführt werden kann.

6. Endoskopisches Nahtligaturinstrument nach den Ansprüchen 1 bis 3, wobei die Verriegelungseinrichtung einen Stopper umfasst, der Folgendes umfasst:
einem Plattenteil mit einem Durchmesser, der größer ist als der Innendurchmesser des Schlauchs, und
eine Verbindungsschleife, die annähernd U-förmig ist und in das Lumen des Schlauchs eingeführt werden kann, wobei ein Ende der Verbindungsschleife einstückig mit einem Teil des Plattenteils verbunden ist, ein mittlerer Teil der Verbindungsschleife durch die Ligaturschlaufe geführt ist und das andere Ende der Verbindungsschleife einst ückig mit einem anderen Teil des Plattenteils verbunden ist.

7. Endoskopisches Nahtligaturinstrument nach einem der Ansprüche 1 bis 3, wobei die Verriegelungseinrichtung einen Stopperring umfasst, der aus einem endlosen Drahtelement besteht, das um das Rohr gewickelt ist, und der Stopperring durch die Verbindungsschlaufe verläuft, während er entlang einer Seitenfläche einer der Außenseiten des Rohrs verläuft, und der Stopperring durch die Ligaturschlaufe verläuft, während er entlang der anderen der Außenseiten des Rohrs verläuft.

8. Endoskopisches Nahtligaturinstrument gemäß einem der Anspr ü che 1 bis 3, wobei die Verriegelungseinrichtung einen mit der Ligaturschlaufe einstückig ausgebildeten Vorsprung umfasst.

9. Endoskopisches Nahtligaturinstrument nach einem der Ansprüche 1 bis 3, wobei die Verriegelungseinrichtung einen Teil mit größerem Durchmesser umfasst, der einstü ckig mit der Ligaturschlaufe ausgebildet ist, wobei der Teil mit größerem Durchmesser einen größeren Durchmesser aufweist als der Durchmesser des anderen Teils der Ligaturschlaufe.

10. Endoskopisches Nahtligaturinstrument nach einem der Ansprüche 1 bis 3, wobei die Verriegelungseinrichtung einen Verriegelungsabschnitt umfasst, der eine größere Größe als der Innendurchmesser des Rohrs aufweist und an einem Endabschnitt der Ligaturschlaufe gegenüber dem Verbindungsteil vorgesehen ist.

## Revendications

1. Outil de ligature de suture endoscopique pour ligaturer une suture suturant une paroi de lumière d'un tractus gastro-intestinal, comprenant :
une boucle de ligature (11a) pour faire passer une suture (4) à travers,
une boucle de liaison (11b) pouvant être connectée à un dispositif de ligature (2),
une partie de connexion (11c) reliant la boucle de ligature et la boucle de liaison,
un tube (12) monté de manière coulissante sur la partie de connexion de sorte que la boucle de ligature soit d'un côté du tube et la boucle de liaison soit du côté opposé du tube, lequel tube est configuré pour être déplacé par coulissement vers le côté de la boucle de ligature ou la boucle de liaison est configurée pour être tirée par rapport au tube de sorte que l'ensemble de la boucle de ligature soit tiré dans une lumière du tube avec la suture, et
un moyen de verrouillage prévu sur la boucle de ligature, la boucle de liaison ou le tube pour verrouiller une partie de la boucle de ligature de sorte que la boucle de ligature ne soit pas libérée de l'intérieur du tube lors du tirage de l'ensemble de la boucle de ligature dans la lumière du tube.

2. Outil de ligature de suture endoscopique selon la revendication 1, dans lequel la partie de connexion 1s tune partie colonnaire, et la boucle de ligature est connectée à une extrémité de la partie de connexion et la boucle de liaison est connectée à l'autre extrémité de la partie de connexion.

3. Outil de ligature de suture endoscopique selon la revendication 1, dans lequel
des parties médianes se faisant face d'un élément en boucle constitué d'un élément filaire sans fin flexible sont mises en contact et le tube est monté à l'extérieur de celles-ci de sorte que la boucle de ligature soit formée par l'élément en boucle d'un côté du tube, et la boucle de liaison soit formée par l'élément en boucle de l'autre côté du tube, et
la partie de connexion 1s tune partie où le tube est monté.

4. Outil de ligature de suture endoscopique selon l'une quelconque des revendications 1 à 3, dans lequel le moyen de verrouillage comprend un élément de sertissage monté sur le tube de sorte qu'une partie de la lumière du tube soit plus étroite.

5. Outil de ligature de suture endoscopique selon l'une quelconque des revendications 1 à 3, dans lequel le moyen de verrouillage comprend une butée comprenant :
une partie plaque ayant un premier trou traversant dans lequel une partie de la boucle de liaison passe de manière coulissante et un second trou traversant dans lequel une autre partie de la boucle de liaison passe, et
une partie saillante formée d'une seule pièce avec la partie plaque sensiblement au centre de la partie plaque tout en faisant saillie vers le côté du tube de sorte que la partie saillante puisse être insérée dans la lumière.

6. Outil de ligature de suture endoscopique selon l'une quelconque des revendications 1 à 3, dans lequel le moyen de verrouillage comprend une butée comprenant :
une partie plaque ayant un diamètre plus grand qu'un diamètre intérieur du tube, et
une boucle de connexion ayant une forme en U et pouvant être insérée dans la lumière du tube, dans laquelle une extrémité de la boucle de connexion est connectée d'une seule pièce à une partie de la partie plaque, une partie intermédiaire de la boucle de connexion passe à travers la boucle de ligature, et l'autre extrémité de la boucle de connexion est connectée d'une seule pièce à une autre partie de la partie plaque.

7. Outil de ligature de suture endoscopique selon l'une quelconque des revendications 1 à 3, dans lequel le moyen de verrouillage comprend un anneau de butée constitué d'un élément filaire sans fin enroulé autour du tube, et l'anneau de butée passe à travers la boucle de liaison tout en longeant une face latérale de l'une des faces extérieures du tube, et l'anneau de butée passe à travers la boucle de ligature tout en longeant l'autre des faces extérieures du tube.

8. Outil de ligature de suture endoscopique selon l'une quelconque des revendications 1 à 3, dans lequel le moyen de verrouillage comprend une partie saillante formée d'une seule pièce avec la boucle de ligature.

9. Outil de ligature de suture endoscopique selon l'une quelconque des revendications 1 à 3, dans lequel le moyen de verrouillage comprend une partie de diamètre plus grand formée d'une seule pièce avec la boucle de ligature, la partie de diamètre plus grand ayant un diamètre plus grand qu'un diamètre de l'autre partie de la boucle de ligature.

10. Outil de ligature de suture endoscopique selon l'une quelconque des revendications 1 à 3, dans lequel le moyen de verrouillage comprend une partie de verrouillage ayant une taille plus grande qu'un diamètre intérieur du tube, et prévue à une extrémité de la boucle de ligature opposée à la partie de connexion.
